(19)

Europäisches
Patentamt

European
Patent Office

Office européen
des brevets

(11) **EP 4 485 211 A1**

(12) **EUROPEAN PATENT APPLICATION**

(43) Date of publication:
**01.01.2025 Bulletin 2025/01**

(21) Application number: **23184718.7**

(22) Date of filing: **11.07.2023**

(51) International Patent Classification (IPC):
**G06F 11/30** (2006.01) **G16H 40/40** (2018.01)

(52) Cooperative Patent Classification (CPC):
**G06F 11/3058; G06F 11/3006; G16H 40/40;
G16H 40/67**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.06.2023 US 202363523383 P**

(71) Applicant: **Koninklijke Philips N.V.
5656 AG Eindhoven (NL)**

(72) Inventors:
• **BHATTACHARYA, Sauvik
Eindhoven (NL)**
• **DEMEWEZ, Tiblets Zeray
Eindhoven (NL)**
• **BARBIERI, Mauro
5656AG Eindhoven (NL)**

(74) Representative: **Philips Intellectual Property &
Standards
High Tech Campus 52
5656 AG Eindhoven (NL)**

(54) **ADAPTIVE AND DYNAMIC HOSTING OF DATA-DRIVEN DIAGNOSTIC CONTENT BY CONTINUOUS MONITORING AND EVALUATION FOR PROACTIVE MAINTENANCE SERVICES**

(57)     A distributed computing network includes an edge computing device and a server computer. The edge computing device receives log data generated by the associated medical device, and is programmed to: perform monitoring of the medical device by analysis of the log data received by the edge computing device; and output alerts generated by the monitoring performed at the edge computing device. The server computer receives the log data from the edge computing device via the Internet, and is programmed to: perform the monitoring of the associated medical device by analysis of the log data received at the server computer; and output alerts generated by the monitoring performed at the server computer. The distributed computing network performs the monitoring of the medical device over time by, at a given time, selecting one of the edge computing device or the server computer to perform the monitoring of the medical device.

200

Fig. 2

**Description**

FIELD OF THE INVENTION

The following relates generally to the medical device maintenance arts, medical imaging device maintenance arts, medical device log file analysis arts, medical predictive maintenance arts, and related arts.

BACKGROUND OF THE INVENTION

Multiple medical imaging systems are typically located at a hospital or site. These systems are communicatively connected to a common electronic network, such as a hospital data network. The medical imaging systems that are located at the same site share the communication bandwidth of a communication channel between the site and the central data processing and storage system. An edge device, comprising a computer servicing the hospital or a portion thereof, e.g., the hospital's radiology department, may collect data from the medical imaging devices at that hospital and then forward the collected data to the central data processing and storage system. This conveniently provides uniformity as the edge devices servicing various hospitals can be standardized, for example provided by the medical imaging devices vendor, so that the central data processing and storage system does not need individualized interfaces for connecting with a wide range of different hospital data networks. A given edge device may be implemented as a physical server or as a virtual machine (VM) running on a server.

Vendors of medical device systems can provide proactive and predictive maintenance services to their customers. More generally, such diagnostic models can generate alerts predicting likely failure events (optionally with a timeframe), and/or can generate alerts about existing problems detected by the models. These services rely on data that comes from medical systems on a regular basis. For this, connectivity of the medical systems is crucial. Moreover, a reliable analytics platform that collects, processes and stores data to build diagnostic models or applications is essential. Typically, these processes take place on the cloud or on-premises, i.e., at the location hosting the medical device.

In a typical arrangement, log data automatically generated by medical imaging devices is uploaded to a server or cloud computing resource maintained by the vendor or other maintenance service provider, and the diagnostic models are also deployed at the server or cloud computing resource. This has advantages such as facilitating updating of the models at a central point, and facilitating communication of alerts to service engineers employed by the vendor or other service provider. However, server or cloud-based models rely heavily on the connectivity of the medical systems, availability, and correctness of the end-to-end data pipeline as well as model deployment infrastructure. Connectivity of medical systems can be hampered by incorrect registration of systems, network interruptions at a customer or vendor site, etc. Furthermore, data pipelines should be robust and reliable to ensure uninterrupted flow of data. Interruptions in data flow disable the proactive services fueled by them, leading possibly to system downtime. Such problems can be remediated to some extent by improving the utilized Internet infrastructure, but the quality of Internet connectivity may be outside the control of both the customer (e.g., hospital) and the vendor or other service provider.

The following discloses certain improvements to overcome these problems and others.

SUMMARY OF THE INVENTION

In one aspect, a system is disclosed for monitoring a medical device. The system includes a distributed computing network comprising an edge computing device and a server computer. The edge computing device is operatively connected to receive log data generated by the associated medical device, and is programmed to: perform monitoring of the medical device by analysis of the log data received by the edge computing device; and output alerts generated by the monitoring performed at the edge computing device. The server computer is configured to receive the log data from the edge computing device via the Internet, and is programmed to: perform the monitoring of the associated medical device by analysis of the log data received at the server computer; and output alerts generated by the monitoring performed at the server computer. The distributed computing network is configured to perform the monitoring of the medical device over time by, at a given time, selecting one of the edge computing device or the server computer to perform the monitoring of the medical device.

In another aspect, at least one non-transitory computer readable medium stores one or more diagnostic models configured to analyze log data generated by a medical device, and instructions readable and executable by at least an edge computing device. The instructions are executable to: monitor the medical device by applying the one or more diagnostic models to generate alerts indicative of problems with the medical device; analyze a plurality of factors to determine whether the monitoring of the medical device should be transferred from the edge computing device to a vendor server; and transfer the monitoring of the medical device from the edge computing device to the vendor server based on the analysis determining that the monitoring of the medical device should be transferred from the edge computing device to the vendor server.

In another aspect, a distributed computing method is disclosed, including: monitoring a medical device by applying one or more diagnostic models to generate alerts indicative of problems with the medical device; analyzing a plurality of factors to determine whether the monitoring of the medical device should be transferred from an edge computing device (14) to a vendor server; and generating an alert indicating that the monitoring of the medical device should be transferred from the edge computing device to the vendor server based on the analysis determining that the monitoring of the medical device should be transferred from the edge computing device to the vendor server.

One advantage resides in leveraging edge computing to ensure uninterrupted availability of analytic applications for monitoring the health of medical imaging devices.

Another advantage resides in dynamically transferring data analytic applications to the customer premises or infrastructure when conditions for hosting them on a centralized (or cloud) platform are not met.

Another advantage resides in dynamically transferring a subset of data analytic applications to the customer premises or infrastructure.

Another advantage resides in dynamically transferring data analytic applications back to the centralized (i.e., cloud) platform when conditions for central hosting are again met.

A given embodiment may provide none, one, two, more, or all of the foregoing advantages, and/or may provide other advantages as will become apparent to one of ordinary skill in the art upon reading and understanding the present disclosure.

BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.

Fig. 1 diagrammatically illustrates an illustrative system analyzing log data in accordance with the present disclosure.
Fig. 2 shows exemplary flow chart operations of the system of Fig. 1.
Fig. 3 shows another embodiment of the system of Fig. 1.

DETAILED DESCRIPTION OF EMBODIMENTS

Remote monitoring of a fleet of imaging devices is performed by a cloud server maintained by the vendor (or other imaging device service provider) which applies diagnostic models to imaging device log data uploaded to the vendor's cloud server to detect existing problems with imaging devices and/or to predict likely failures in support of proactive maintenance. While this arrangement usually works well, it relies on continuous Internet connectivity between the cloud server and the hospital (or other imaging device site). However, interruptions can arise due to loss of Internet connectivity between a given hospital and the cloud server handling the remote monitoring. Such an interruption, if sufficiently extended, can result in missing important predictive maintenance alerts.

In some installations an edge computing device is maintained locally at the hospital to improve interfacing between the hospital and the vendor's cloud server. In this arrangement, the log data is transferred to the edge computing device which then transfers the log data to the cloud server. The edge computing device may be a dedicated physical server computer or may be implemented as a virtual machine (VM) on the hospital's information technology (IT) server computer. This has advantages, such as providing for data latency in transfer of the log data (e.g., the data can be buffered at the edge computing device before being transferred to the cloud server), and simplifying the interfacing as the edge computing device provides a consistent (e.g. same) interface with the cloud server across the fleet of imaging devices.

The disclosed system leverages the edge computing device to also provide a temporary backup capable of performing the remote monitoring locally on the edge computing device at times when connectivity with the cloud server is lost or deemed insufficiently reliable. One or more factors can be analyzed to determine when it would be advantageous or necessary to transfer the remote monitoring from the vendor's cloud server to the local edge computing device. This analysis is done in the cloud and/or at the edge computing device. Advantageously, the edge computing device is already configured to receive the log data from the imaging devices at the hospital or other customer served by the edge device, and so that data is available for analysis at the edge computing device.

There are significant benefits to primarily hosting the diagnostic analyses at the vendormaintained cloud-based server. The edge computing device generally has less computing power than a cloud server, and hence the edge computing device may in some instances be unable to implement the full breadth of diagnostic models that can be hosted at the vendor's cloud server. Furthermore, the alerts generated by the diagnostic analyses are generally intended for action service engineers with the expertise for interpreting the alerts and performing any requisite maintenance. Still further, hosting the diagnostic analyses at the vendor's server facilitates centralized updates to the diagnostic models.

Hence, while in disclosed approaches the edge computing device provides a backup for performing the diagnostic analyses, this functionality is advantageously transferred back to the vendor cloud server when reliable Internet connectivity is restored. In some embodiments, the analysis to determine when the monitoring should be transferred back to the cloud server can be performed at the local edge computing device or at the cloud server, or by a joint decision such as having the local server send a notice to the cloud indicating it is ready to hand the remote monitoring back to the vendor's cloud server and then having the cloud server make the final decision.

To implement the approach, a copy of the remote monitoring software (e.g., the predictive models) is installed at the local edge computing device. This may be difficult to do in a just-intime (JIT) paradigm as the transfer of remote monitoring may be in response to a loss of Internet connectivity, thus making the JIT installation from the cloud difficult or impossible. Hence, the remote monitoring software in some embodiments is installed together with other software of the imaging device(s) being monitored, if the edge computing device has sufficient memory. Updates to the remote monitoring software are then pushed along with updates to the imaging device software to keep the remote monitoring capability at the local edge computing device up to date. It should be noted that the copy of the remote monitoring software (e.g., diagnostic models) that is installed at the local edge computing device may be a subset or a copy of other reduced-functionality of the remote monitoring software deployed at the vendor's cloud server. For example, a subset or a copy of reduced-functionality may be designed for execution on the edge device which has more limited processing power compared with the vendor's cloud server.

In some embodiments, one or more alerts can be generated at the local edge computing device. If the alert is of a type that can be handled by a local biomed and the hospital has such a suitably trained biomed on staff, then the alert could be handled locally, so that the vendor is never involved. If the alert cannot be handled locally then it is forwarded to the vendor (via the Internet or telephonically if there is no Internet connectivity). In this case the alert should be given high priority at the vendor since the issue is known to the customer. (Also, even if the alert is handled at the hospital, the alert along with a summary of the action taken locally at the hospital may still be forwarded to the vendor so that the vendor can update its maintenance records for the medical device appropriately).

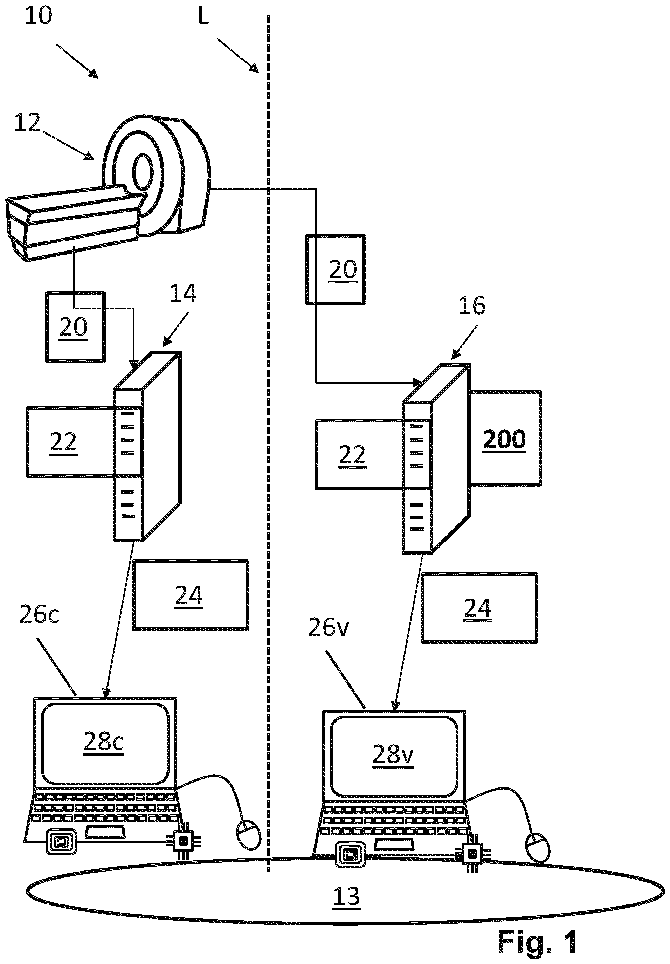

With reference to Fig. 1, an illustrative servicing support system 10 for supporting a service engineer in servicing a device 12 (e.g., a medical imaging device as shown in Fig. 1- also referred to as a medical device, an imaging device, imaging scanner, and variants thereof) is diagrammatically shown. By way of some non-limiting illustrative examples, the medical imaging device under service may be a magnetic resonance imaging (MRI) scanner, a computed tomography (CT) scanner, a positron emission tomography (PET) scanner, a gamma camera for performing single photon emission computed tomography (SPECT), an interventional radiology (IR) device, or so forth. (More generally, the disclosed approach can be applied in conjunction with any type of computerized device or machine that automatically generates log data that are analyzed by predictive models to predict component failures, e.g., the approach could be applied to a commercial airliner, radiation therapy device, a cardo ship, an industrial robot, or so forth). In addition, although only a single device 12 is shown in Fig. 1, the support system 10 can analyze log data from a fleet of devices 12 (i.e., more than one device).

As shown in Fig. 1, the servicing support system 100 includes, or is accessible by, a distributed computing network comprising one or more distributed computers electronically connected to each other via an electronic network 13 (e.g., a wired connection, a local area network (LAN), the Internet, wireless Wi-Fi or 4G/5G interface or the like for connection to the Internet and/or an intranet, and so forth).

The distributed computing network includes one or more local customer computers 14 (i.e., "edge devices") configured to analyze log data from the device 12. A number of edge devices 14 corresponds to the number of devices 12 in the fleet. For example, the edge device(s) 14 can be disposed adjacent to, or in a same building as, the corresponding device(s) 12. A single edge device computer 14 is shown in Fig. 1, and can comprise a server computer (e.g., single server computer, or a server cluster, or a cloud computing resource comprising ad hoc-interconnected server computers, or so forth). The edge computing device 14 could be a virtual machine (VM) running on a physical hospital server computer or the like. Whether physical or a VM, the term edge computing device 14 or edge device 14 is used herein. The distributed computing network also includes a vendor server computer 16. Each edge computing device 14 is programmed to receive log data automatically generated by the imaging device 12 and to transfer the log data to the vendor server computer 16. The vendor's server computer 16 can comprise a server computer as shown, or can be a server cluster, or a cloud computing resource comprising ad hoc-interconnected server computers. Whether implemented as single server computer, a cluster of server computers, or a cloud computing resource, the term server computer 16 is used herein to encompass all such embodiments. The distributed computing network 14, 16 thus comprises the edge computing device 14 and the server computer 16.

The medical device(s) 12 is configured to generate a dataset of log data 20 for analysis by the edge computing device 14 and the vendor server computer 16. For example, the log data 20 may include diverse information generated by sensors of the illustrative medical imaging device 12 (e.g., temperature readings for various components and so forth), fault indicators produced by components of the imaging device 12, information on imaging scans run by the imaging device 12 and other usage data, and/or so forth. The log data 20 can comprise (or at least be capable of being represented as) log data strings generated by the device 12, although other data formats are contemplated. The log data 20 for a particular device 12 can be transferred to the corresponding edge computing device 14, which then transfers the log data 20 to the vendor's sever computer 16. In addition, as shown in Fig. 1, each of the computers 14, 16 can be located in geographically diverse locations (as indicated by the dashed lines L in Fig. 1), typically with the edge computing device **14** located at the hospital or other medical facility hosting the medical device **12** and the server computer **16** being located remotely from the hospital, e.g. at the vendor's site if it is self-hosted or at a server farm if the server computer **16** is implemented as a cloud computing resource hosted by a third-party commercial provider.

Each of the edge computing device **14** and the vendor's server computer **16** also comprise a non-transitory computer readable medium configured to store one or more diagnostic models **22** configured to analyze the log data **20**. The diagnostic models **22** can provide various types of diagnoses. For example, the diagnostic models **22** can include predictive models that predict a likely failure (typically with a timeframe to fail) of a component of the imaging device **12**. As a nonlimiting illustrative example, in the case of a CT scanner a predictive model can predict the remaining life of the X-ray tube based on logged usage hours and/or other information such as tube current and voltage measurements. Alerts generated by a predictive model can be used for timely scheduling of preventative maintenance, such as replacing an X-ray tube before it actually fails. As another example, the diagnostic models **22** can include detection models that detect existing problems with the imaging device **12**. For example, a model can detect a component that is running hot, i.e., at too-high temperature, as measured by a temperature sensor of the component. Based on such an alert, a service engineer can schedule an inspection or, if the problem is sufficiently serious, contact the hospital to recommend stopping usage of the medical device **12** until the problem is fixed. These are merely some nonlimiting illustrative examples of suitable diagnostic models **22**.

For example, the vendor server computer(s) **16** are configured to also receive the log data **20** from the device(s) **12**, perform monitoring of the medical device **12** by analysis of the log data **20**, and output one or more alerts **24** generated by the monitoring. For example, the edge computing device(s) **14** are in communication with a corresponding vendor electronic processing device **26v** (e.g., a computer, a workstation, a smartphone, a tablet, and so forth) operable by an employee of the vendor (e.g., a remote service engineer (RSE), a field service engineer (FSE), and so forth). A graphical user interface (GUI) **28v** is displayed on a display device of the electronic processing device **26v** to display the alert(s) **24**. The alert(s) **24** can be indicative of a performance of the device **12** based on the analysis of the log data **20**.

In addition, the edge computing device(s) **14** are configured to receive the log data **20** from the device(s) **12**, perform monitoring of the medical device **12** by analysis of the log data **20**, and output one or more alerts **24** generated by the monitoring. For example, the edge computing device(s) **14** are in communication with a corresponding local customer electronic processing device **26c** (e.g., a computer, a workstation, a smartphone, a tablet, and so forth) operable by an employee of the customer (e.g., a technician and so forth). A local graphical user interface (GUI) **28c** is displayed on a display device of the electronic processing device **26c** to display the alert(s) **24**. The alert(s) **24** can be indicative of a performance of the device **12** based on the analysis of the log data **20**. In some examples, the edge computing device **14** is further programmed to classify each alert **24** as to whether the alert **24** requires remote assistance, and to additionally output alerts **24** classified as requiring remote assistance to the vendor server computer **16**.

The distributed computing network **14, 16** is configured to perform the monitoring of the medical device **12** over time by, at a given time, selecting one of the edge computing device **14** or the server computer **16** to perform the monitoring of the medical device **12**. In one example, this selection is based on at least on a quality metric of Internet connectivity between the server computer **16** and the edge computing device **14** at the given time. In another example, this selection is based on an indication generated by the edge computing device **14** of whether the edge computing device **14** is ready to perform the monitoring of the medical device **12**. In another example, if the edge device **14** abruptly loses all connectivity with the server computer **16** then the edge device **14** can select itself (i.e., select the edge device **14**) to perform the monitoring until its connection with the server computer **16** is re-established.

At some point in time, the server computer **16** is configured to transfer software executable to perform the analysis of the log data **20** received by the edge computing device **14** from the server computer **16** to the edge computing device **14**. This could in some instances be done at the time the selection is made to have the edge computing device **14** temporarily take over the monitoring. However, in practice this may be impractical, since the decision to transfer monitoring may be made in response to detection of unreliable connectivity between the edge device **14** and the server computer **16** (at which point the unreliable connection may preclude transferring the software). Hence, in some embodiments the software transfer is done at an earlier time, such as when the edge computing device **14** is installed. To transfer the software from the server computer **16** to the edge computing device **14**, the software is optionally encrypted at the server computer **16** before transfer. The encrypted software is transferred from the server computer **16** to the edge computing device **14**, and the encrypted software is decrypted and deployed at the edge computing device **14**.

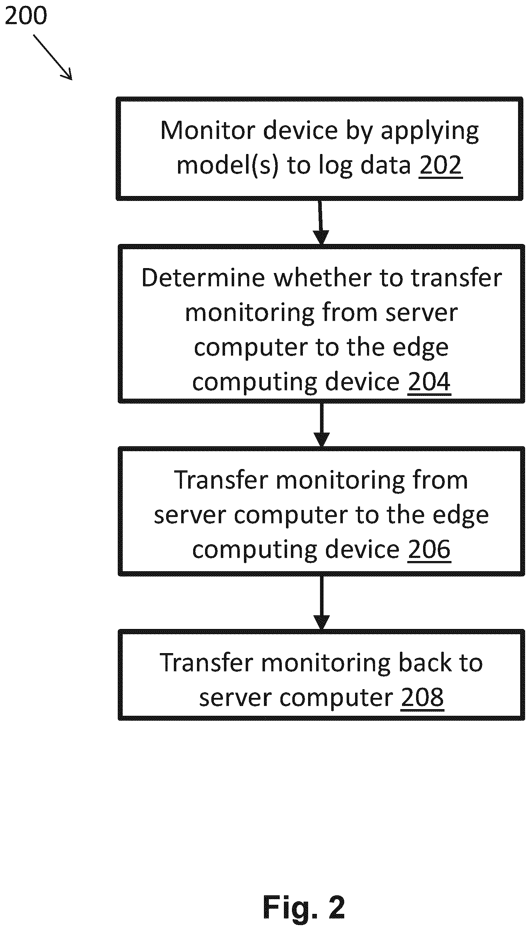

With continuing reference to Fig. 1 and further reference to Fig. 2, an illustrative embodiment of the method **200** is diagrammatically shown as a flowchart. In some examples, the method **200** may be performed at least in part by cloud processing.

To begin the method **200**, at an operation **202**, the server computer **16** is configured to monitor the medical device **12** by applying the diagnostic model(s)

22 to the log data **20** received at the server computer **16** from the medical device **12** via the edge device **14** to generate the alerts **24** indicative of problems with the medical device **12**. These alerts will generally be displayed at the vendor's GUI **26v** (see Fig. 1) for review and possible action by a service engineer or other agent of the vendor.

At an operation **204**, the distributed computing network **14, 16** is configured to analyze a plurality of factors to determine whether the monitoring of the medical device **12** should be transferred from the server computer **16** to the edge computing device **14**. The factors can include, for example, one or more of a status of connectivity between the edge device **14** and server computer **16**, a presence of suitable infrastructure (e.g. the edge computing device **14** with a copy of the diagnostic models **22**) at the edge device **14**, a number of technicians at the hospital or other site of the edge computing device **14**, and so forth. As previously mentioned, the selection operation **204** is performed by the distributed computing network **14, 16**, and may in a given embodiment be performed entirely by the server computer **16**, or entirely by the edge device **14**, or may be performed cooperatively by both devices **14** and **16** (e.g., the server computer **16** may send a request to take over monitoring to the edge device **14** and the edge device **14** then accepts the request to complete the selection of the edge device **14**). The selection **204** may also in some embodiments be performed at a given time by different devices, e.g. normally the server computer **16** may make the selection **204** but if there is an abrupt and complete loss of Internet connectivity then the selection may be made by the (now isolated) edge computing device **14** on its own. The factors for making the selection **204** can also include an availability of the log data **20** and a presence of historical log data for the medical device **12**.

At an operation **206**, the distributed computing network **14, 16** is configured to transfer the monitoring of the medical device **12** from the server computer **16** to the edge computing device **14** based on the analysis operation **204** determining that the monitoring of the medical device **12** should be so transferred.

After such a transfer, the distributed computing network **14, 16** continues to analyze the factors to determine when the server computer **16** should resume the monitoring of the medical device **12**. At an operation **208**, the monitoring of the medical device **12** is transferred from the edge computing device **14** back to the server computer **16**. In some examples, the server computer **16** can generate and transmit a notification from the server computer **16** to the edge computing device **14** indicating readiness of the server computer **16** to resume the monitoring.

EXAMPLES

The system **10** is configured to address the dependency of proactive and predictive maintenance services on uninterrupted connectivity and data availability by using edge computing. Edge computing is a technique that enables to deploy models on a device or in the vicinity of a device. The system **10** is configured to regularly assess a cloud deployment platform of diagnostic models **22** using dynamic measures that assist in deciding whether to keep the model **22** in the cloud or temporarily move them to the edge computer **14**. This ensures continuity of providing proactive and predictive services. When the situation at hand improves, the model **22** is moved back to the vendor server **16** to take advantage of centralized hosting on the cloud.

The dynamic measures are defined based on the characteristics of the customer and the content of the model as described below. These measures can include, for example, a status of the connectivity of the device **12** at the customer's site, an IT infrastructure to enable alerting to the hospital personnel, a biomed setup (i.e., "Does the customer have biomeds that can follow up when an alert is generated by a diagnostic model at the customer's site?"), and so forth. The models **22** can include a dependency on immediate data availability (i.e., "Does the model need real-time data, or can it deal with a latency of e.g. a few days?"), and a dependency on historical data (i.e., "Does the model need to use a (large) set of historical data, or can it make a prediction based on relatively little data?").

In case of edge-based model, a biomed gets notification whenever an alert **24** is generated. The biomed reports to the vendor that assign the right personnel to resolve the issue. The vendor pays a fee to the customer for every alert reported by a biomed. Cloud-based models are managed in the existing process where an alert is reported directly to vendor personnel.

Leveraging edge computing for maintenance services offers the possibility of applying proactive and predictive maintenance services to a wider set of devices including the ones that have connectivity issue due to poor internet connection. Consequently, it reduces data transfer, processing, and storage costs. Moreover, it minimizes onsite diagnostic cost and avoids planned maintenance services. It also allows a vendor to offer such services to smaller hospitals who do not have the necessary IT and network infrastructure in place to ensure uninterrupted flow of data to the central (vendor) cloud. It also allows extending such services to hospitals that do not wish to, or are restricted by regulation or policy (e.g., military hospitals), connect their devices to the Internet.

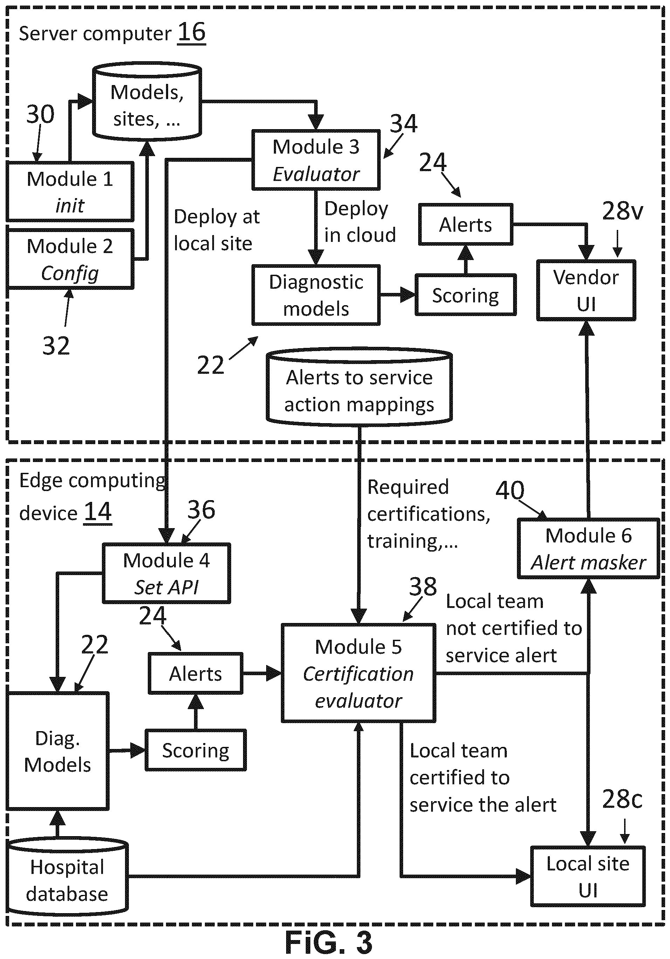

Fig. 3 shows another embodiment of the system **10**. Fig. 3 shows a detailed overview of the various modules and data sources involved in the system **10**. It consists of multiple parameters, as well as functional modules that use these parameters as input to execute the invention. Let $M = \{m_1, m_2, ..., m_i\}$, a set of $i$ proactive and predictive **22**, and let $S = \{s_1, s_2, ..., s_j\}$, a set of $j$ sites hosting (medical) systems (e.g., a number of edge device computers **14**) which are in scope of the set of models *M* **22** apply.

The top-level computer **18** is configured to analyze a function $C = \{(c, u, v, t)_1, (c, u, v, t)_2, ..., (c, u, v, t)_k\}$, a set of *k* quadruples defined per site $s \in S$, capturing characteristics of the site (i.e., the edge computer **14**) and whether it is suitable for hosting the models **22**. *c* is a characteristic or metric that is evaluated when deciding whether to host m in the vendor server **16** or transfer it to the local infrastructure of the edge computer **14** at *s*. *u* is the last known value of the characteristic c for the site *s*. *v* is the ideal value of the characteristic c recorded over all sites in the set *S*. *t* is a noise tolerance factor that allows to ignore minor fluctuations in the value of *u*, when comparing it against the ideal value *v*. In a first example, a status of the connectivity of the device at a specific customer site is analyzed , in which *c* is an "Average log file download rate for systems at site", $u = 75\%$, $v = 98\%$, $t = 1$ representing that there is a 1% chance of the value of *u* being an outlier or too noisy to be compared with *v*, making a resampling necessary. In a second example, a suitability of local IT infrastructure for hosting models **22** is analyzed, in which c is a "Number of multi-threading workstations with more than 15GB of RAM", $u = 2$, $v = 1$, and $t = N/A$. In a third example, a determination is made as to "Does the customer have biomedical engineers that can follow up on alerts **24** generated by the models **22**?", in which *c* is a "Number of biomedical engineers locally available per monitored system", $u = 1$, $v = 2$, $t = N/A$.

Let $\mathcal{M} = \{(\mu, p)_1, (\mu, p)_2, ..., (\mu, p)_l\}$, a set of *l* tuples for each model $m \in M$, capturing characteristics of the model **22**. $\mu$ is a dependency or need of the model **22**, (e.g., "Longest tolerable time difference in days between current date and date of the *latest* available data for the model to evaluate" (real time data needed? or "Lowest tolerable time difference in days between current date and date of the *oldest* available data" (how much historical data is needed to reinforce model prediction?), and so forth). *p* is the value for each $\mu$. Exemplary values corresponding to the above examples of $\mu$ could be "1", "365" etc.

Fig. 3 also shows different phases of the method **200**. In an initialization phase, a Module 1 "Init" **30** allows a user to define and initialize values of all parameters, including models **22**, sites, characteristics etc. This module **30** also allows adding new models **22**, sites, or characteristics during operation. A Module 2 "Config" **32** allows a user to define how frequently the invention evaluates where to host models **22**. This evaluation is done for all models **22** in *M*. For example, setting this to 1 day, 12:00AM ensures that every midnight the site and model characteristics $C$, $\mathcal{M}$ for all sites $s \in S$ and all models $m \in M$ are evaluated using the latest known data to decide if the model **22** should continue to be hosted in the vendor server **16**, or be offloaded to the site of the edge computer **14**.

In a decision phase, a module 3 "Evaluator" **34** is implemented. At a frequency and time of day configured by module 3 "Config", this module **34** chooses whether to continue hosting a model **22** centrally in the cloud vendor server **16** to offload it to its local site of the edge computer **14**, by evaluating site and model characteristics $C$, $\mathcal{M}$ for all sites $s \in S$ and all models $m \in M$. It does this for all sites $s \in S$ and all models $m \in M$. Note that site characteristics $C$ capture live data as well. For every model $m \in M$ and site $s \in S$, the evaluator module **34** consists of a function $char(m, s) = \{C, \mathcal{M}\}$ which returns the characteristics $C$ defined for m and s, and m's own characteristics $\mathcal{M}$. The evaluator module **34** also consists of a function $d(m, s, char(m, s)) \in \{0,1\}$ that returns the evaluation decision of whether the model m should be offloaded to the site *s* (output 1) or not (output 0).

In a transfer phase, depending on the result of the decision phase **222** (via the output of the function $d(m, s, char(m, s))$), the model **22** in the form of its associated data analytics functions are packaged, encrypted and offloaded to the local infrastructure of the site of the edge computer **14**.

In a "**Preparation stage for offloaded deployment" phase**, a module 4 "Set_API" 36 allows a user at a given site $s \in S$ to define the input and output APIs for the model **22** when it is hosted locally at the edge computing device **14**. This ensures that the model execution is agnostic to and integrates easily into the local infrastructure. The input API can then be used by the local infrastructure to pass required input parameters to the model **22**, and the output API allows the model output to be consumed by local monitoring or response tools.

At an execution phase, a Module 5 "Certification validator" **38** is configured to check whether the local operational team of the site where the model **22** has been offloaded to (e.g., biomedical engineers at the hospital site) has required certification for direct servicing of an alert **24**. This can be implemented by maintaining a mapping of model and alert categories and their corresponding service actions, to the required certifications. If the result of the check indicates that the local team is trained and certified, the full unfiltered alert **24** with all details is sent via the configured output API to the local operational team's infrastructure (e.g., a hospital dashboard). A Module 6 "Alert masker" **40** receives the output of the "certification validator" **38**, and masks/removes alert details if the local operational team does not have required training. It then sends this masked alert via the configured output API 36 to the local operational team, and allows them to forward the alert with priority to the manufacturer.

In some embodiments, the "Config" module **32** allows a user to set evaluation frequencies per model **22**. Thus, instead of a global frequency and time of day when all models $m \in M$ for all sites $s \in S$ are evaluated, the user can define a set of *i* evaluation frequencies for each model 22. The default evaluation frequency for any model **22** is twice its execution frequency. This ensures that the decision of whether to transfer it to a different hosting location is taken and executed well in advance of execution of the model.

In some embodiments, the maximum amount of time allowed to transfer a model to a different hosting location, e.g., offloaded from the vendor server **16** to the edge computing device **14** or vice versa, is upper bounded to a fixed default value. (The characteristics of) any new model **22** or site that is added to the sets $M$ and $S$ by the "Init" module to verify whether the time required for the model **22** to be offloaded to that site, or transferred to the cloud from the site within the maximum limit. This embodiment can lead to performance improvements in situations where models execute frequently, e.g., multiple times a day. Upper bounding the acceptable transfer time allows the benefits of dynamic transfer of hosting without interrupting the model execution.

In some embodiments, depending on the capabilities of the site to which a model **22** is offloaded (represented by the site characteristics parameter

$$C = \{(\varsigma, u, v, t)_1, (\varsigma, u, v, t)_2, \dots, (\varsigma, u, v, t)_k\})$$

and the needs of the model **22** (represented by the model characteristics $\mathcal{M} = \{(\mu, p)_1, (\mu, p)_2, \dots, (\mu, p)_l\}$), only a subset of the data analytics functions critical to the functioning of the model are offloaded to the local site. For example, if the site characteristics indicate that the local infrastructure is of low specifications, then only the functions required to generate the alert **24** itself, and primary output parameters about the alert and the system may be transferred to the local site. Additional functions that generate secondary output parameters may be skipped.

A non-transitory storage medium includes any medium for storing or transmitting information in a form readable by a machine (e.g., a computer). For instance, a machine-readable medium includes read only memory ("ROM"), solid state drive (SSD), flash memory, or other electronic storage medium; a hard disk drive, RAID array, or other magnetic disk storage media; an optical disk or other optical storage media; or so forth.

The methods illustrated throughout the specification, may be implemented as instructions stored on a non-transitory storage medium and read and executed by a computer or other electronic processor.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

**Claims**

1. A system **(10)** for monitoring an associated medical device **(12)**, the system comprising:
   a distributed computing network **(14, 16)** comprising an edge computing device **(14)** and a server computer **(16)**, wherein:

   the edge computing device **(14)** is operatively connected to receive log data **(20)** generated by the associated medical device, and is programmed to:

   perform monitoring of the medical device by analysis of the log data received by the edge computing device; and
   output alerts **(24)** generated by the monitoring performed at the edge computing device;

   the server computer **(16)** is configured to receive the log data from the edge computing device via the Internet **(13)**, and is programmed to:

   perform the monitoring of the associated medical device by analysis of the log data received at the server computer; and
   output alerts generated by the monitoring performed at the server computer; and

   the distributed computing network is configured to perform the monitoring of the medical device over time by, at a given time, selecting one of the edge computing device or the server computer to perform the monitoring of the medical device.

2. The system (10) of claim 1, wherein the distributed computing network **(14,** 16) is configured to select the one of the edge computing device **(14)** or the server computer **(16)** to perform the monitoring of the associated medical device **(12)** at a given time based at least on a quality metric of Internet connectivity between the server computer and the edge computing device at the given time.

3. The system **(10)** of claim 2, wherein the distributed computing network **(14,** 16) is configured to select the one of the edge computing device **(14)** or the server computer **(16)** to perform the monitoring of the associated medical device **(12)** at a given time further based on an indication generated by the edge computing device of whether the edge computing device is ready to perform the monitoring of the medical device.

4. The system **(10)** of any one of claims 1-3, wherein the distributed computing network **(14, 16)** is configured to program the edge computing device **(14)** to perform the monitoring of the associated medical device **(12)** by:
transferring software executable to perform the analysis of the log data **(20)** received by the edge computing device from the server computer **(16)** to the edge computing device.

5. The system **(10)** of claim 4, wherein the transferring of the software from the server computer **(16)** to the edge computing device **(14)** includes:

encrypting the software at the server computer wherein the encrypted software is transferred from the server computer to the edge computing device; and
decrypting the encrypted software at the edge computing device.

6. The system **(10)** of any one of claims 1-5, wherein:

the server computer **(16)** is further programmed to output the alerts **(24)** generated by the monitoring performed at the server computer to an associated remote user interface **(28v);** and
the edge computing device **(14)** is further programmed to output the alerts generated by the monitoring performed at the edge computing device to an associated local user interface **(28c)** different from the associated remote user device.

7. The system **(10)** of claim 6, wherein the edge computing device **(14)** is further programmed to classify each alert **(24)** generated by the monitoring performed at the edge computing device as to whether the alert requires remote assistance and to additionally output alerts classified as requiring remote assistance to the associated remote user interface **(28v).**

8. At least one non-transitory computer readable medium **(14, 16)** storing:
one or more diagnostic models **(22)** configured to analyze log data generated by a medical device **(12);**
instructions readable and executable by at least an edge computing device **(14)** to:

monitor the medical device by applying the one or more diagnostic models to generate alerts **(24)** indicative of problems with the medical device;
analyze a plurality of factors to determine whether the monitoring of the medical device should be transferred from the edge computing device to a vendor server **(16);** and
transfer the monitoring of the medical device from the edge computing device to the vendor server based on the analysis determining that the monitoring of the medical device should be transferred from the edge computing device to the vendor server.

9. The at least one non-transitory computer readable medium **(14, 16)** of claim 8, wherein the instructions further include
transferring the monitoring of the medical device from the vendor server **(16)** back to the edge computing device **(14)** in response to the analysis no longer determining that the monitoring of the medical device **(12)** should be transferred from the edge computing device to the vendor server.

10. The at least one non-transitory computer readable medium **(14, 16)** of claim 9, wherein the instructions further include
generating and transmitting a notification from the edge computing device **(14)** to the vendor server **(16),** the notification being indicative of a readiness of the edge computing device to resume the monitoring.

11. The at least one non-transitory computer readable medium **(14, 16)** of any one of claims 8-10, wherein the edge computing device **(14)** is maintained by a customer of the medical device **(12)** and configured to store and implement the one or more diagnostic models **(22)** to analyze log data **(20)** generated by the medical device.

12. The at least one non-transitory computer readable medium **(14, 16)** of any one of claims 8-11, wherein the vendor server **(16)** is maintained by a vendor of the medical device **(12)** and configured to store a copy of the one or more diagnostic models **(22).**

13. The at least one non-transitory computer readable medium **(14, 16)** of any one of claims 8-12, wherein the plurality of factors includes one or more of a status of connectivity of the edge computing device **(14)** to the medical device **(12),** a presence of infrastructure at a site of the vendor server **(16),** and a number of technicians at the site of the edge computing device.

14. The at least one non-transitory computer readable medium **(14, 16)** of claim 13, wherein the plurality of factors further includes an availability of log data **(20)** generated by the medical device **(12)** and a presence of historical log data for the medical device.

15. The at least one non-transitory computer readable medium **(14, 16)** of any one of claims 8-14, wherein the instructions further include:
generating an alert **(24)** to be handled by a medical professional at a site of the edge computing device **(14).**

Fig. 1

200

Monitor device by applying
model(s) to log data 202

Determine whether to transfer
monitoring from server
computer to the edge
computing device 204

Transfer monitoring from
server computer to the edge
computing device 206

Transfer monitoring back to
server computer 208

**Fig. 2**

**FIG. 3**

Europäisches
Patentamt

European
Patent Office

Office européen
des brevets

# EUROPEAN SEARCH REPORT

Application Number

EP 23 18 4718

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/046037 A1 (RAMESH MANEESHA VINODINI [IN] ET AL) 14 February 2019 (2019-02-14) | 1-15 | INV. G06F11/30 G16H40/40 |
| Y | * abstract * * paragraphs [0006], [0045], [0060] - [0067], [0073], [0105] * ----- | 1-15 | |
| Y | US 2022/215948 A1 (BARDOT DAWN [US]) 7 July 2022 (2022-07-07) * abstract * * paragraphs [0063], [0632] * ----- | 1-15 | |
| A | EP 4 187 550 A1 (KONINKLIJKE PHILIPS NV [NL]) 31 May 2023 (2023-05-31) * the whole document * ----- | 1-15 | |

|  |
|---|
| TECHNICAL FIELDS SEARCHED (IPC) |
| G06F G16H |

The present search report has been drawn up for all claims

| Place of search | Date of completion of the search | Examiner |
|---|---|---|
| Munich | 10 July 2024 | Leuridan, Koen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 18 4718

This annex lists the patent family members relating to the patent documents cited in the above-mentioned European search report.
The members are as contained in the European Patent Office EDP file on
The European Patent Office is in no way liable for these particulars which are merely given for the purpose of information.

10-07-2024

| Patent document cited in search report | Publication date | Patent family member(s) | | Publication date |
|---|---|---|---|---|
| US 2019046037 A1 | 14-02-2019 | NONE | | |
| US 2022215948 A1 | 07-07-2022 | AU | 2022205948 A1 | 27-07-2023 |
| | | CA | 3207484 A1 | 14-07-2022 |
| | | CN | 116963662 A | 27-10-2023 |
| | | DE | 112022000538 T5 | 09-11-2023 |
| | | EP | 4271256 A1 | 08-11-2023 |
| | | IL | 304194 A | 01-09-2023 |
| | | JP | 2024504060 A | 30-01-2024 |
| | | KR | 20230142486 A | 11-10-2023 |
| | | US | 2022215948 A1 | 07-07-2022 |
| | | US | 2022399112 A1 | 15-12-2022 |
| | | WO | 2022150523 A1 | 14-07-2022 |
| EP 4187550 A1 | 31-05-2023 | NONE | | |

EPO FORM P0459

For more details about this annex : see Official Journal of the European Patent Office, No. 12/82